# EUROPEAN PATENT APPLICATION

(11) **EP 4 002 377 A1**
(43) Date of publication of application: **25.05.2022**
(21) Application number: 21210131.5
(22) Date of filing: 24.11.2021
(51) Int. Cl.: G16H 10/20, G16H 10/60, G16H 20/30, G16H 20/60, G16H 50/70, G16H 80/00

(54) **PERSONALIZED MEAL DIET AND EXERCISE PROVIDING METHOD USING INTEGRATED HEALTH INFORMATION AND SERVICE SYSTEM**

(30) Priority: 24.11.2020 KR 20200158477
(71) Applicant: NGeneBio Co., Ltd., Guro-gu Seoul 08390 (KR)
(72) Inventor: YANG, Sung Woo, 14563 Gyeonggi-do (KR); YU, Hyo Jin, 18483 Gyeonggi-do (KR); SONG, Young Kyu, 06697 Seoul (KR); JUNG, Soon Woo, 06981 Seoul (KR); JUNG, Jin Hyuck, 13578 Gyeonggi-do (KR)
(74) Representative: Ostertag & Partner Patentanwälte mbB

(57) **Abstract**

Disclosed is a method of recommending a diet and an exercise using integrated health information, the method including allowing a service server to receive genome information of a specific individual, allowing the service server to receive clinical information of the specific individual, allowing the service server to receive lifestyle habit information of the specific individual, allowing the service server to input the genome information, the clinical information, and the lifestyle habit information to a pre-trained learning model, and allowing the service server to generate diet information for the specific individual with reference to a diet database on the basis of an output value output by the learning model and generate exercise information for the specific individual with reference to the exercise database on the basis of the output value.

## Description

### CROSS-REFERENCE TO RELATED APPLICATION

This application claims the benefit under 35 U.S.C. § 119(a) of Korean Patent Application No. 10-2020-0158477, filed on November 24, 2020, in the Korean Intellectual Property Office, the entire disclosure of which is incorporated herein by reference for all purposes.

### BACKGROUND

### 1. Field of the Invention

The following description relates to a technique for recommending a customized diet and exercise using composite health information.

### 2. Discussion of Related Art

A variety of information is used to analyze an individual's health state. An individual's genome information can be said to be a representative indicator for analyzing a health state. Furthermore, recently, human intestinal microorganisms have been attracting attention as a target for analyzing an individual's health state. Various studies are being conducted on various diseases related to the human microbiome.

### SUMMARY OF THE INVENTION

The following description is intended to provide a system that provides diet information and exercise information that can enhance a specific individual's health state by considering not only human genome information but also a wide variety of data that may be related to the individual's health.

According to an aspect of the present disclosure, there is provided a method of recommending a diet and an exercise using integrated health information, the method including allowing a service server to receive genome information of a specific individual, allowing the service server to receive clinical information of the specific individual, allowing the service server to receive lifestyle habit information of the specific individual, allowing the service server to input the genome information, the clinical information, and the lifestyle habit information to a pre-trained learning model, and allowing the service server to generate diet information for the specific individual with reference to a diet database on the basis of an output value output by the learning model and generate exercise information for the specific individual with reference to the exercise database on the basis of the output value.

According to another aspect of the present disclosure, there is provided a system for recommending a diet and an exercise using integrated health information, the system including a user terminal configured to transmit survey information and lifestyle habit information of a specific individual and receive diet information and exercise information, a genome information server configured to provide genome information for the specific individual, a medical information server configured to provide first medical information of the specific individual and second medical information of a family member of the specific individual, a diet database configured to store the diet information according to a health state, an exercise database configured to store exercise information according to a health state, and a service server configured to input the survey information, the genome information, the first medical information, the second medical information, and the lifestyle habit information to a pre-trained learning model to generate an output value for the specific individual, configured to generate the diet information for the specific individual with reference to the diet database on the basis of the output value, and configured to generate the exercise information for the specific individual with reference to the exercise database on the basis of the output value.

### BRIEF DESCRIPTION OF THE DRAWINGS

FIG 1 is an example of a diet and exercise recommendation system;
FIG 2 is an example of a diet and exercise recommendation process;
FIG. 3 is an example of a process of processing collected data;
FIG. 4 is an example of a health information output process using a learning model; and
FIG. 5 is an example of a diet and exercise execution process corresponding to an individual's health state.

Throughout the drawings and the detailed description, the same reference numerals refer to the same elements. The drawings may not be to scale, and the relative size, proportions, and depiction of elements in the drawings may be exaggerated for clarity, illustration, and convenience.

### DETAILED DESCRIPTION OF EXEMPLARY EMBODIMENTS

As the following description may be variously modified and have several example embodiments, specific embodiments will be shown in the accompanying drawings and described in detail below. It should be understood, however, that there is no intent to limit the following description to the particular forms disclosed, but on the contrary, the following description is to cover all modifications, equivalents, and alternatives falling within the spirit and scope of the invention.

In addition, the terms such as "first," "second," "A," and "B" may be used to describe various elements, but these elements are not limited by these terms. These terms are used to only distinguish one element from another element. For example, a first element may be called a second element, and a second element may also be called a first element without departing from the scope of the following description. The term "and/or" means any one or a combination of a plurality of related items.

It should be understood that singular forms are intended to include plural forms unless the context clearly indicates otherwise, and it should be further understood that the terms "comprise," "include," or "have" as used herein specify the presence of stated features, numerals, steps, operations, elements, components, or a combination thereof but do not preclude the presence or addition of one or more other features, numerals, steps, operations, elements, components, or combinations thereof.

Prior to a detailed description of the drawings, it should be clarified that division of components in the present specification is performed merely based on main functions performed by the respective components. That is, two or more components which will be described later may be integrated into a single component or, alternatively, a single component may be divided into two or more components depending on subdivided functions. Further, it is apparent that each of the components, which will be described later, may additionally perform some or all of the functions performed by other components in addition to main functions performed thereby, and some of the main functions performed by the respective components may be shared with other components and may be performed.

In addition, the respective steps of the above method may be performed in a sequence different from a described sequence unless the context clearly defines a specific sequence. That is, the respective steps may be performed in the same order as described, substantially simultaneously, or in reverse order.

A host is an object that microorganisms inhabit. Here, the host is assumed to be a human.

A sample refers to a specimen extracted from the host. The sample is a specimen for determining host genome information or disease information, such as blood or tissue. Also, the sample may be a specimen capable of identifying intestinal microorganisms, such as feces.

Genome information refers to genome data of a specific individual or entity. The genome information may be acquired using various techniques. The entity basically includes humans, animals, plants, microorganisms, and the like. For example, the genome information may include nucleotide sequences, gene expression data, genetic variation with standard genome data, DNA methylation, and the like which are obtained from deoxyribonucleic acid (DNA), ribonucleic acid (RNA), or protein from cells, tissues, etc. Typically, the genome information may be gene information obtained using next-generation sequencing (NGS).

The genome information may include epigenome information. The epigenome information refers to factors that affect gene expression and includes information related to DNA methylation, histone modification, microRNA, and the like.

A microbiome is defined as any microbial communities such as bacteria, viruses, and fungi that inhabit and coexist in a human body at the individual level and genetic information of these microbial communities. In general, the human microbiome includes information about microorganisms that inhabit the human intestine, oral cavity, skin, and the like. The human microbiome includes information such as the whole metagenome along with the 16s rRNA of the microorganisms. The amount of 16s rRNA can be determined by performing metagenome analysis on various samples from humans. Metagenome analysis may be performed using various bioinformatics tools related to the metagenome analysis. The purpose of the analysis is to determine the function and distribution of microorganisms derived from a user's body, usually by measuring the amount of microorganisms. The human microbiome includes operational taxonomic unit (OTU) information obtained from raw information for 16s rRNA analysis. Furthermore, whole-genome sequencing is a method of decoding the entire nucleotide sequence of a sample, and the result of the analysis includes both of human genetic information and microbial genetic information. Therefore, a researcher can check genetic information of a microbial community by extracting only the microbial genetic information (the filtering of the human genetic information).

An individual's genome information may be used to include a host's genome information and microbiome information.

Clinical information may include various items such as biometric information, medical treatment information, and prescription information about an individual (patient). For example, clinical information may include an individual's body information (age, gender, race, height, weight, etc.), examination information (blood test results, X-ray images, CT images, MRI images, electrocardiogram tests, genomic analysis results, etc.), diagnosis results (health states, disease information, etc.).

Meanwhile, the clinical information may include questionnaire information and survey information for measuring personal health.

Lifestyle habit information refers to information related to lifestyle habits. For example, the lifestyle habit information may include (i) physical activity information, (ii) food intake information, (iii) exercise information, and the like. The physical activity information may include sleep time, wake up time, work time, rest time, and the like. The food intake information may include a daily calorie intake, the amount of intake nutrients, and the like. The exercise information may include information on whether to exercise, the intensity of exercise, and the like. The lifestyle habit information may be collected through a smart device, an Internet of Things (IoT) device, a wearable device, etc.

Each of the genome information, the clinical information, and the lifestyle habit information is an indicator related to an individual's health. A variety of information associated with an individual's health is called integrated health information. The integrated health information includes the above-described genome information, clinical information, and lifestyle habit information.

The technique to be described below can estimate an individual's health state using a learning model. The learning model refers to a machine learning model. The learning model includes various types of models. For example, the learning model may be a decision tree, a random forest, a K-nearest neighbor (KNN), a naive Bayes, a support vector machine (SVM), an artificial neural network, and the like. As for artificial neural networks, deep neural networks (DNNs) are attracting attention recently. DNNs include a convolutional neural network (CNN), a recurrent neural network (RNN), a restricted Boltzmann machine (RBM), a deep belief network (DBN), a generative adversarial network (GAN), relation networks (RLs), and the like.

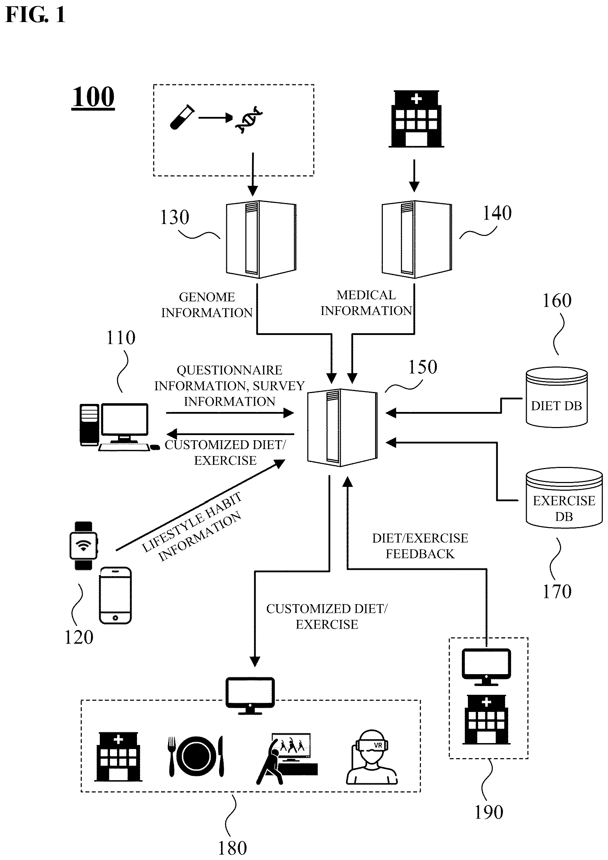

FIG. 1 is an example of a diet and exercise recommendation system 100. A service server 150 estimates the health state of a specific individual and recommends a customized diet and exercise on the basis of the health state.

The service server 150 collects individual information in order to construct integrated health information of an individual whose health state is to be checked.

A user terminal 110 transmits questionnaire information or survey information for a specific individual. The questionnaire information and the survey information include items that a service provider wants to check in advance.

A wearable device 120 transmits lifestyle habit information of a specific individual. Various devices such as a smart device and an IoT device may collect and transmit lifestyle habit information. Meanwhile, the user terminal 110 may collect information collected by peripheral devices and transmit the lifestyle habit information.

A genome information server 130 transmits genome information of a specific individual. The genome information server 130 may analyze a sample of the specific individual to generate the genome information. The description of a process of collecting samples and analyzing genome will be omitted. The genome information may be composite information including a host's whole genome and whole metagenome or sequence information of a specific target and a specific marker (16s rRNA Sequencing). Furthermore, the genome information may include information on an epigenome, a transcriptome, and a proteome.

A medical information server 140 transmits clinical information of a specific individual. A medical institution may transmit clinical information including a specific individual's medical treatment result, test result, disease information, and the like. In some cases, the medical information server 140 may also transmit genome information of a specific individual. Also, the medical information server 140 may transmit questionnaire information or survey information collected during a medical treatment process.

The service server 150 receives survey information, lifestyle habit information, genome information, clinical information, etc. and uniformly processes the received information to generate integrated health information. The service server 150 inputs the integrated health information to a pre-trained learning model and estimates health state information of the specific individual. Various models such as a statistical analysis model and a neural network may be used as the learning model.

The learning model generates an output value related to an individual's health state. The learning model may output feature information for an individual's health state. The learning model may be a model for extracting feature information from the integrated health information. Alternatively, the learning model may generate a deviation value for an individual's health state by comparing the input information to a reference value on the basis of the integrated health information.

Based on an output value of the learning model, the service server 150 may determine diet information related to the output value with reference to a diet database (DB) 160. The diet DB 160 holds a health state and diet information matched to the health state. Alternatively, the diet DB 150 may hold an output value of the learning model and diet information matched to the output value. The diet information may include menus, restaurant information, and the like that can promote health according to a specific individual's health state.

Based on an output value of the learning model, the service server 150 may determine exercise information associated with the output value with reference to an exercise DB 170. The exercise DB 170 holds a health state and exercise information matched to the health state. Alternatively, the exercise DB 170 may hold an output value of the learning model and exercise information matched to the output value. The exercise information refers to information on an exercise that can promote health according to a specific individual's health state. The exercise information may include an exercise type, exercise content, an exercise program, etc.

The service server 150 delivers the diet information and exercise information determined according to an individual's health state to a service terminal 180. The service terminal 180 is a terminal used by a subject receiving information. The service terminal 180 may include various terminals, such as a hospital terminal, a restaurant terminal, a fitness center terminal, and a personal terminal. Meanwhile, the service terminal 180 may be of various types, such as a computer device, a smart device, a smart TV, a game console, and a virtual reality (VR) device. Simply, the service terminal 180 may be a device configured to receive and output diet information and health information. Furthermore, the service terminal 180 may be a device configured to provide an exercise program related to the exercise information. Also, the service terminal 180 may be a smart refrigerator or a food cooking device that displays food or ingredient information in relation to the diet information.

An expert terminal 190 reviews an individual's health state and diet information/exercise information corresponding to the personal health state, which are delivered by the service server 150. The expert terminal 190 may determine whether the recommended diet information/exercise information is appropriate and transmit feedback on the determination to the service server 150. The service server 150 may transmit, to the service terminal 180, diet information/exercise information modified in consideration of the feedback of the expert terminal 190. Also, the service server 150 may update the learning model by using the feedback of the expert terminal 190. The update of the learning model refers to a procedure in which more appropriate output values are generated by changing parameter values applied to the model.

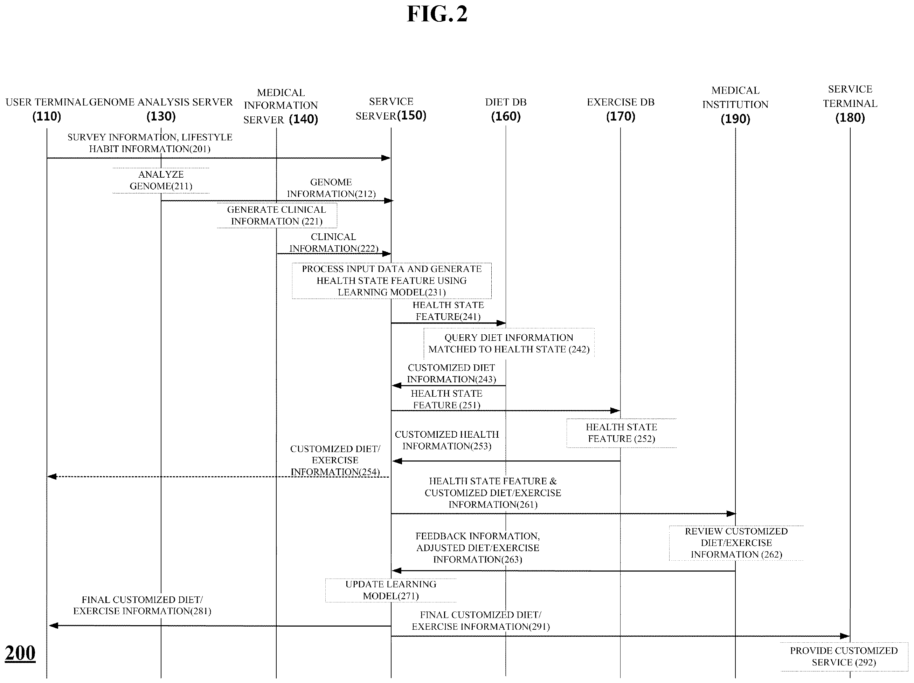

FIG. 2 is an example of a diet and exercise recommendation process 200.

A user terminal 110 transmits survey information and lifestyle habit information to a service server 150 (201). The lifestyle habit information may be collected and delivered by a separate wearable device or the like.

The survey information may include at least a plurality of items from the item group consisting of stress resilience, inflammatory response, neurological function, boost energy-level, blood glucose response to meals, body mass composition, sleep, nutrient absorption, physical fatigue, and food allergy. The survey information may include information about an individual's stress and emotional state.

The lifestyle habit information may include information such as sleep amount, sleep time, sleep pattern, exercise amount, exercise time, walking time, walking distance, and the like. Furthermore, the lifestyle habit information may include personal sound information. The sound information may include voice, snoring, etc.

The genome analysis server 130 may perform a genome analysis on an individual (211). Alternatively, the genome analysis server 130 may receive a result obtained by a genome analysis apparatus. The genome analysis server 130 transmits genome information to the service server 150 (212). The genome information includes host genome information, epigenome information, and microbiome information.

A medical information server 140 generates the individual's clinical information (221). The clinical information includes the individual's medical treatment information, test data, test history, etc. Furthermore, the clinical information may include medical treatment information, test information, test histories, etc. for specific individual's family members. This is because a family history can be an indicator for determining health state information.

The service server 150 processes received lifestyle habit information, survey information, genome information, and clinical information into input data that can be analyzed by a learning model and generates a health state feature using the learning model (231).

The service server 150 may transmit the health state feature to a diet DB 160 (241), query the diet DB 160 about diet information matched to the health state (242), and receive customized diet information from the diet DB 160 (243).

The service server 150 may transmit the health state feature to an exercise DB 170 (251), query the diet DB 170 about exercise information matched to the health state (252), and receive customized exercise information from the exercise DB 170 (253).

The service server 150 may instantly transmit customized diet information and exercise information to the user terminal 110 (254).

The service server 150 may transmit the health state feature and the customized diet/exercise information to the expert terminal 190 (261). The expert terminal 190 generates a result of reviewing whether the diet information and/or the exercise information is appropriate with reference to the received health state feature (262). The service server 150 may receive, from the expert terminal 190, feedback information on the customized diet information and exercise information (263). The service server 150 may receive, from the expert terminal 190, diet information and exercise information to which a recommendation has been added or whose content has been adjusted (263).

The service server 150 may update its learning model with reference to the feedback received from the expert terminal 190 (271).

The service server 150 may transmit, to the user terminal 110, diet information and exercise information to which an expert's recommendation has been added or whose content has been adjusted (281).

The service server 150 may transmit, to the service terminal 180, diet information and exercise information to which an expert's recommendation has been added or whose content has been adjusted (291). The service terminal 180 may provide a customized service to a user according to the received diet information and exercise information (292).

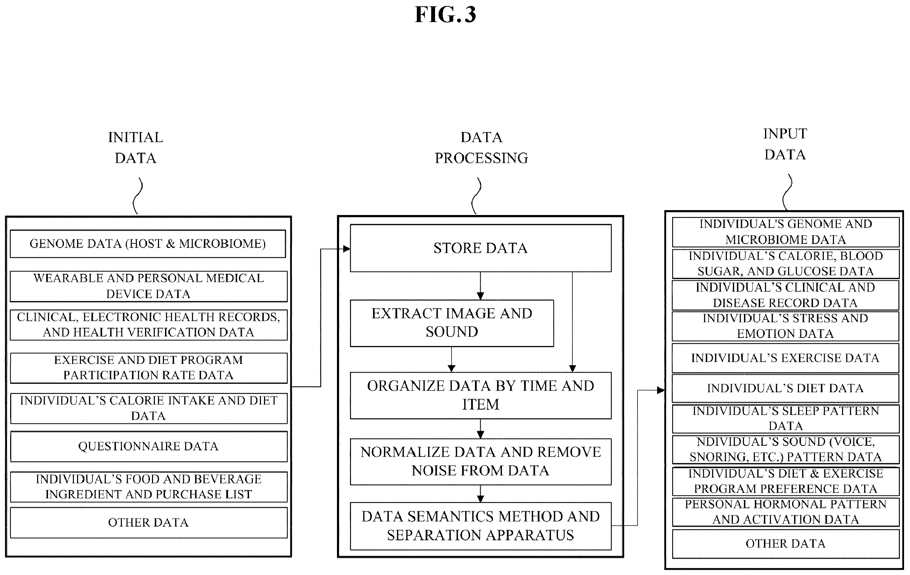

FIG. 3 is an example of a process of processing collected data. FIG. 3 corresponds to a process of the service server 150 preprocessing received information to generate input data that can be used for a learning model.

Initial data is data delivered by a user terminal, a wearable device, a medical information server, a genome analysis server, etc.

Genome information includes host genome information and a microbiome. The host genome information may include epigenome information.

Wearable device and personal medical device data is data collected by an individual and may include lifestyle habit data and clinical information.

Clinical, electronic health record, and health verification data is data generated by a medical institution.

Exercise and diet program participation rate data refers to the degree to which an individual participates in a provided exercise and diet program. The participation rate data may be transmitted to a user terminal. Alternatively, the participation rate data may be transmitted to a separate server. The participation rate data may be used to determine an individual's preference for a particular exercise or diet.

Personal calorie intake and diet data is information on consumed food and calories. The calorie and diet data may be transmitted by the user terminal. Alternatively, the calorie and diet data may be transmitted by a separate server.

Questionnaire data is response data prepared for an individual's questionnaire. The questionnaire data may be transmitted through a user terminal, a medical institution terminal, etc.

An individual's food and beverage ingredient possession and purchase list is information about foods that have been possessed or purchased by the individual. The food and beverage ingredient possession and purchase list may be transmitted by a user terminal. Alternatively, the food and beverage ingredient possession and purchase list may be transmitted by a home appliance or a separate server. The food and beverage ingredient possession and purchase list may be used to determine an individual's preference.

The service server 150 receives and stores various kinds of initial data. The service server 150 may separately extract image and sound data rather than text from the data. The service server 150 may organize the initial data on a time basis. Also, the service server 150 may organize data for each item of the data.

The service server 150 may uniformly normalize the data and remove noise contained in the data.

The service server 150 may classify and separate the data using a semantics method on the basis of the normalized data.

Through this process, input data that can be used in a learning model is generated. As the input data, (i) personal genome data and microbiome data, (ii) personal calorie, blood glucose, and glucose data, (iii) personal clinical and disease record data, (iv) personal stress and emotion data, (v) personal exercise amount data, (vi) personal diet data, (vii) personal sleep pattern data, (viii) personal sound (voice, snoring, etc.) pattern data, (ix) personal diet & exercise program preference data, (x) personal hormonal pattern and activity data and the like may be calculated. FIG. 3 illustrates a portion of the available data.

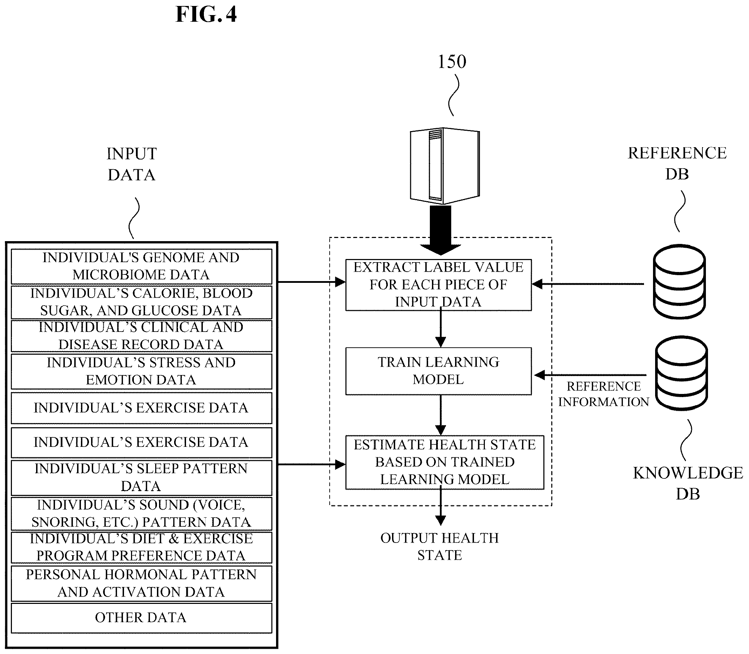

FIG. 4 is an example of a health information output process using a learning model. The input data is data generated through the process shown in FIG. 3.

The service server 150 may process the input data according to each item. Alternatively, the service server 150 may form the input data into information in one integrated form. For example, when a learning model uses vector data in a matrix form, the service server 150 may form the input data in a matrix form according to a certain rule. Furthermore, the service server 150 may form individual input data according to a data type.

The input data may vary depending on the number of input data types illustrated in FIG. 4. When the learning model has the same type as a neural network model, the learning model may have different structures depending on the input data. When the input data is in one matrix form, an input layer may be one layer in which a matrix is received and processed. Furthermore, when the input data is formed as individual input data according to an information type, the input layer may have a plurality of layers corresponding to the number of pieces of input data. Alternatively, when the plurality of pieces of input data are formed as one matrix, the entire input data may be composed of a plurality of matrices. In this case, features extracted by the plurality of input layers may be integrated and processed in a hidden layer or a fully connected layer.

The learning model may have a different model learning process depending on its type. A supervised learning model needs label values for input data and output values corresponding to the input data. The service server 150 may provide a label value for a set of input data. For example, the service server 150 may extract a health state matched to the input data from a reference DB and provide a label value.

Meanwhile, the service server 150 may compare a standard value for the same item to the input data. The service server 150 may extract a standard value for the input data from the reference DB and determine a difference between the input data and the standard value. For example, the service server 150 may perform comparisons between personal sleep data and standard sleep data, between personal clinical data and standard clinical data (a healthy person), between personal food and beverage ingredient data and standard food and beverage ingredient data, between personal microbiome data and standard microbiome data, and between personal biomarker and genome mutation data and may check a deviation of an individual to be analyzed for each item. The service server 150 may use the difference between each item of the input data and a standard value as an input value of the learning model. In this case, the service server 150 may provide health state information corresponding to the deviation of the corresponding item as a label value.

As described above, the service server 150 may input the input data of various items itself or the deviation between the input data and the standard value as input data and may train the learning model using a label value prepared in advance.

Furthermore, the service server 150 may verify a result inferred by the learning model based on the input data with reference to a knowledge DB that holds expert or universal knowledge. The service server 150 may constantly update the learning model on the basis of the verification result.

When the learning model is trained, the service server 150 may enter input data of an individual to be analyzed into the learning model and thus estimate the health state of the corresponding individual.

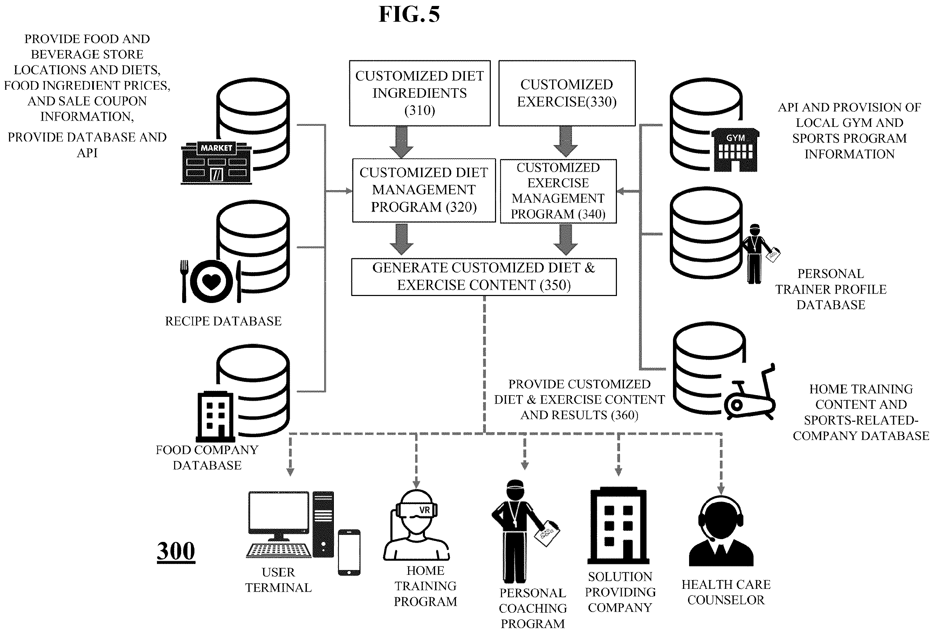

FIG. 5 is an example of a diet and exercise execution process 300 corresponding to an individual's health state.

A service server 150 generates customized diet information according to a personal health state. The service server 150 determines diet ingredients capable of improving health with reference to a diet DB on the basis of an individual's health state (310). The service server 150 may generate a program for customized diet management on the basis of the determined diet ingredients (320).

It is assumed that the service server 150 has a built-in and accessible DB capable of providing food and beverage store locations, menus, food ingredient prices, sale coupons, and the like and has a related API installed therein. In this case, the service server 150 may generate information on food, menus, and stores matched to the diet ingredients determined in operation 310 (320).

The service server 150 may generate cooking menu information that can be used for cooking on the basis of the diet ingredients determined in operation 310 with reference to a recipe DB (320). The service server 150 may find a matching food company on the basis of the diet ingredients determined in operation 310 with reference to a food company DB and generate store information (320).

The service server 150 generates customized exercise information according to a personal health state (330). The service server 150 may generate an exercise management program capable of improving health with reference to an exercise DB on the basis of an individual's health state (340).

It is assumed that the service server 150 has a built-in and accessible DB capable of providing community athletic facilities and sports program information and has a related API installed therein. In this case, the service server 150 may generate exercise program information matched according to the exercise information determined in operation 330 (340). The service server 150 may generate trainer information matched according to the exercise information determined in operation 330 with reference to a personal trainer profile DB (340). The service server 150 may generate trainer information matched according to the exercise information determined in operation 330 with reference to a personal trainer profile DB (340). The service server 150 may generate matching sports company information according to the exercise information determined in operation 330 with reference to a sports-related company DB (340).

The service server 150 may generate customized diet and exercise content (350). The service server 150 may provide the generated customized diet and exercise content to individuals or service providers (360). Through a user terminal, an individual may check diet and exercise content and determine diet and exercise. An individual may execute a home training program using a virtual reality (VR) device or the like. A trainer may check a customer's diet and exercise content and provide a program that fits the information. A solution provider company may recommend a program tailored to an individual according to the individual's diet and exercise content. A health consulting company may consult an individual with reference to the individual's diet and exercise content.

The above description enhances an individual's health by using genome information, clinical information, and lifestyle habit information in combination. The above description integrates information managed individually by medical institutions or IoT devices and recommends a customized diet and exercise through a learning model that receives the integrated information. The above description provides information for a customized food service provider and a customized exercise service provider.

Also, the above-described learning model-based health state estimation method, customized diet information recommendation method, and customized exercise information recommendation method may be implemented as a program (or an application) including an executable algorithm that can be executed in a computer. The program may be stored and provided in a transitory or non-transitory computer-readable medium.

The non-transitory computer-readable medium refers to a medium that semi-permanently stores data and is readable by a device rather than a medium that temporarily stores data such as a register, a cache, and a memory. Specifically, the above-described various applications or programs may be stored and provided in a non-transitory computer-readable medium such as a compact disc (CD), a digital versatile disc (DVD), a hard disk, a Blu-ray disc, a Universal Serial Bus (USB), a memory card, a read-only memory (ROM), a programmable read-only memory (PROM), an erasable PROM (EPROM), an electrically EPROM (EEPROM), or a flash memory.

A transitory computer-readable medium refers to various RAMs such as a static RAM (SRAM), a dynamic RAM (DRAM), a synchronous DRAM (SDRAM), a double data rate SDRAM (DDR SDRAM), an enhanced SDRAM (ESDRAM), a SyncLink DRAM (SLDRAM), and a Direct Rambus RAM (DRRAM).

While this disclosure includes specific examples, it will be apparent after an understanding of the disclosure of this application that various changes in form and details may be made in these examples without departing from the spirit and scope of the claims and their equivalents. The examples described herein are to be considered in a descriptive sense only, and not for purposes of limitation. Descriptions of features or aspects in each example are to be considered as being applicable to similar features or aspects in other examples. Suitable results may be achieved if the described techniques are performed in a different order, and/or if components in a described system, architecture, device, or circuit are combined in a different manner, and/or replaced or supplemented by other components or their equivalents. Therefore, the scope of the disclosure is defined not by the detailed description, but by the claims and their equivalents, and all variations within the scope of the claims and their equivalents are to be construed as being included in the disclosure.

## Claims

1. A method of recommending a diet and an exercise using integrated health information, the method comprising:
receiving, by a service server, genome information of a specific individual;
receiving, by the service server, clinical information of the specific individual;
receiving, by the service server, lifestyle habit information of the specific individual;
inputting, by the service server, the genome information, the clinical information, and the lifestyle habit information to a pre-trained learning model; and
generating, by the service server, diet information for the specific individual with reference to a diet database on the basis of an output value output by the learning model and generate exercise information for the specific individual with reference to the exercise database on the basis of the output value,
wherein the genome information comprises host genome information, microbiome genome information, and host epigenome information.

2. The method of claim 1, wherein
the service server is configured to individually input the genome information, the clinical information, and the lifestyle habit information to a plurality of input ports of the learning model or
the service server is configured to integrate the genome information, the clinical information, and the lifestyle habit information into one piece of information and input the information to the learning model in a two-dimensional (2D) matrix form.

3. The method of claim 1, wherein the clinical information includes survey data input by the specific individual, medical information of the specific individual, and medical information of a family member of the specific individual.

4. The method of claim 3, wherein the survey data at least includes data on a plurality of items among stress resilience, inflammatory response, nervous system function, boost energy-level, blood sugar response to a meal, body mass composition, sleep, nutrient absorption, physical fatigue, and food allergy.

5. The method of claim 1, wherein
the lifestyle habit information is collected through an Internet of Things (IoT) device, and
the lifestyle habit information includes sleep information and sound information.

6. The method of claim 1, wherein
the service server further receives a food purchase history and an exercise program participation history of the specific individual from a separate server, and
the service server generates the diet information by further reflecting a food preference of the specific individual on the basis of the food purchase history and generates the exercise information by further reflecting an exercise preference of the specific individual on the basis of the exercise program participation history.

7. A system for recommending a diet and an exercise using integrated health information, the system comprising:
a user terminal configured to transmit survey information and lifestyle habit information of a specific individual and receive diet information and exercise information;
a genome information server configured to provide genome information for the specific individual;
a medical information server configured to provide first medical information of the specific individual and second medical information of a family member of the specific individual;
a diet database configured to store the diet information according to a health state;
an exercise database configured to store exercise information according to a health state; and
a service server configured to input the survey information, the genome information, the first medical information, the second medical information, and the lifestyle habit information to a pre-trained learning model to generate an output value for the specific individual, configured to generate the diet information for the specific individual with reference to the diet database on the basis of the output value, and configured to generate the exercise information for the specific individual with reference to the exercise database on the basis of the output value,
wherein the genome information comprises host genome information, microbiome genome information, and host epigenome information.

8. The system of claim 7, wherein the survey data at least includes information on a plurality of items among stress resilience, inflammatory response, nervous system function, boost energy-level, blood sugar response to a meal, body mass composition, sleep, nutrient absorption, physical fatigue, and food allergy.

9. The system of claim 7, wherein the service server further receives a food purchase history and an exercise program participation history of the specific individual from a separate server, generates the diet information by further reflecting a food preference of the specific individual on the basis of the food purchase history, and generates the exercise information by further reflecting an exercise preference of the specific individual on the basis of the exercise program participation history.

10. The system of claim 7, further comprising an expert terminal configured to transmit an expert's review opinion on the diet information and the exercise information,
wherein the service server adds the review opinion to the diet information and the exercise information and transmits the review opinion to the user terminal.

11. The system of claim 7, further comprising a medical institution terminal configured to transmit an expert's review opinion on the diet information and the exercise information,
wherein the service server updates parameters of the learning model on the basis of the review opinion.
